# FASCICULE DE BREVET EUROPEEN

(11) **EP 3 431 034 B1**
(45) Date de publication et mention de la délivrance du brevet: **26.02.2020**
(21) Numéro de dépôt: 17181702.6
(22) Date de dépôt: 17.07.2017
(51) Int. Cl.: A61C 1/14, A61B 17/16

(54) **SOUS-ENSEMBLE POUR PIÈCE À MAIN DE SOIN DENTAIRE OU DE CHIRURGIE, PIÈCE À MAIN DE SOIN DENTAIRE OU DE CHIRURGIE, ET PROCÉDÉ DE MONTAGE ASSOCIÉ**
UNTERELEMENT FÜR ZAHNÄRZTLICHES ODER CHIRURGISCHES HANDSTÜCK, ZAHNÄRZTLICHES ODER CHIRURGISCHES HANDSTÜCK UND ENTSPRECHENDES MONTAGEVERFAHREN
SUBASSEMBLY FOR DENTAL OR SURGICAL HANDPIECE, DENTAL OR SURGICAL HANDPIECE, AND ASSOCIATED ASSEMBLY METHOD

(43) Date de publication de la demande: 23.01.2019
(73) Titulaire: Bien-Air Holding SA, 2504 Bienne (CH)
(72) Inventeur: Juillerat, Sébastien, 2740 Moutier (CH)
(74) Mandataire: BOVARD AG

(56) Documents cités:
- EP-A1- 1 378 207
- FR-A1- 2 873 566
- JP-A- 2002 095 679

## Description

### Domaine technique de l'invention

La présente invention se rapporte au domaine des instruments et appareils pour les praticiens dans le secteur médical, tels que les dentistes et les chirurgiens. Plus précisément, elle concerne un sous-ensemble pour pièce à main de soin dentaire ou de chirurgie, une pièce à main de soin dentaire ou de chirurgie, ainsi qu'un procédé de montage du sous-ensemble susmentionné.

### État de la technique

Les dentistes, les chirurgiens et d'autres praticiens dans le secteur médical ont recours à divers instruments et appareils. Lorsqu'il est doté d'un outil, un instrument ou appareil pour praticien dans le secteur médical peut être conçu de manière que cet outil soit amovible notamment afin de pouvoir être remplacé par un autre outil. Lorsque tel est le cas, un bouton-poussoir peut être prévu et permettre d'appliquer une commande manuelle de déverrouillage de l'outil.

Dans chacune des demandes de brevet EP 1 378 207, DE 432 44 93 et EP 1 733 696, il est proposé une pièce à main à usage dentaire ou chirurgical, qui comporte un tel bouton-poussoir. Cette pièce à main comporte également un arbre creux associé à des moyens d'entraînement. La queue d'un outil de travail, notamment un outil de fraisage, peut être montée dans cet arbre creux et y être déverrouillée par un dispositif que le bouton-poussoir sert à actionner.

Dans la pièce à main proposée dans la demande de brevet EP 1 378 207 parmi celles mentionnées ci-dessus, le bouton-poussoir est retenu par des griffes qui se déforment élastiquement pour laisser passer un rebord du bouton-poussoir lors du montage de celui-ci. Le bouton-poussoir de la pièce à main de EP 1 378 207 ne peut pas être démonté sauf à détériorer les griffes.

Dans chacune des pièces à main proposées dans les demandes de brevet DE 432 44 93 et EP 1 733 696 mentionnées précédemment, le bouton-poussoir est retenu par une bague extérieure vissée sur un corps d'assemblage renfermant le mécanisme intérieur de la pièce à main. Cette bague extérieure peut s'accrocher et même occasionner des blessures. De plus, un interstice circonférentiel débouchant à l'extérieur est présent là où un bord de la bague extérieure porte sur le corps d'assemblage. La présence de cet interstice nuit à un nettoyage et une désinfection efficaces de l'extérieur de la pièce à main.

### Exposé sommaire de l'invention

L'invention a pour but de permettre, entre autres, qu'un bouton-poussoir équipant une pièce à main de soin dentaire ou de chirurgie soit démontable et qu'aucun interstice débouchant à l'extérieur ne résulte de la présence de moyens retenant ce bouton-poussoir.

Selon l'invention, ce but est atteint grâce à un sous-ensemble pour pièce à main de soin dentaire ou de chirurgie. Ce sous-ensemble comporte un bouton-poussoir comprenant une surface d'appui adaptée pour recevoir une pression manuelle à même d'actionner le bouton-poussoir en coulissement selon une direction de coulissement. Le sous-ensemble selon l'invention comporte en outre une pièce de retenue du bouton-poussoir, ainsi qu'un dispositif à baïonnette qui est à même de maintenir assemblés le bouton-poussoir et la pièce de retenue. Dans ce dispositif à baïonnette, le bouton-poussoir comporte plusieurs portions d'accrochage décalées angulairement entre elles autour d'un axe parallèle à la direction de coulissement et la pièce de retenue comporte plusieurs butées de fin de course à même de stopper les portions d'accrochage vers l'extérieur, ainsi que plusieurs passages ouverts latéralement qui passent entre les butées de fin de course, qui mènent derrière ces butées de fin de course et que les portions d'accrochage sont à même d'emprunter pour venir derrière les butées de fin de course.

Le sous-ensemble défini ci-dessus peut incorporer une ou plusieurs autres caractéristiques avantageuses, isolément ou en combinaison, en particulier parmi celles précisées ci-après.

Avantageusement, le bouton-poussoir comporte une première portion où se trouve la surface d'appui, le bouton-poussoir comportant une deuxième portion qui est une jupe fermée d'un côté par la première portion, la première portion et la jupe délimitant un creux dans lequel pénètre au moins une partie de la pièce de retenue et dans lequel se trouvent les butées de fin de course et les portions d'accrochage. Lorsque tel est le cas, l'aire de la surface d'appui peut être maximisée sans que soient modifiées les dimensions extérieures de la pièce à main.

Avantageusement, la pièce de retenue est également une pièce de guidage du bouton-poussoir selon la direction de coulissement.

Avantageusement, le sous-ensemble comporte au moins un ergot que porte une première pièce parmi le bouton-poussoir et la pièce de retenue, la deuxième pièce parmi le bouton-poussoir et la pièce de retenue comportant au moins une glissière de guidage de l'ergot selon la direction de coulissement.

Avantageusement, la pièce de retenue comporte la glissière qui possède une extrémité obstruée par une des butées de fin de course, tandis qu'un des passages fait partie de la pièce de retenue et débouche dans la glissière, et que l'ergot fait partie du bouton-poussoir, forme une des portions d'accrochage et est à même d'emprunter le passage débouchant dans la glissière.

Avantageusement, la pièce de retenue comporte la glissière qui possède une extrémité obstruée par une des butées de fin de course, l'ergot étant mobile en translation dans la glissière entre une première extrémité et une deuxième extrémité se trouvant au-dessus de la première extrémité et au niveau de l'extrémité obstruée de la glissière, un des passages faisant partie de la pièce de retenue et débouchant dans la glissière, à distance de la première extrémité de la course maximale, tandis que l'ergot fait partie du bouton-poussoir, forme une des portions d'accrochage et est à même d'emprunter le passage débouchant dans la glissière. Lorsque tel est le cas, il n'y a aucun risque que le bouton-poussoir puisse être démonté quand il est enfoncé complètement sous l'action d'une pression manuelle. Il en résulte une sécurité accrue rendant plus fiable l'utilisation du bouton-poussoir.

Avantageusement, la pièce de retenue comporte la glissière qui possède une extrémité obstruée par une des butées de fin de course, l'ergot étant mobile en translation dans la glissière entre une première extrémité et une deuxième extrémité opposée se trouvant au-dessus de la première extrémité et située au niveau de l'extrémité obstruée de la glissière, un des passages faisant partie de la pièce de retenue et débouchant dans la glissière, à distance de la première extrémité et de la deuxième extrémité de la glissière, tandis que l'ergot fait partie du bouton-poussoir, forme une des portions d'accrochage et est à même d'emprunter le passage débouchant dans la glissière. Lorsque tel est le cas, il n'y a aucun risque que le bouton-poussoir puisse être démonté aussi bien quand il est enfoncé complètement sous l'action d'une pression manuelle que quand il est dans sa position la plus vers l'extérieur, par exemple sous l'action d'un organe élastique de rappel de ce bouton-poussoir vers l'extérieur. Il en résulte une sécurité accrue rendant plus fiable l'utilisation du bouton-poussoir.

Inversement, l'ergot également peut faire partie de la pièce de retenue et former une des butées de fin de course, le bouton-poussoir comprenant la glissière. Lorsque tel est le cas, la glissière possède avantageusement une extrémité obstruée par une des portions d'accrochage, une course maximale de l'ergot dans la glissière comprenant une première extrémité et une deuxième extrémité se trouvant en arrière de la première extrémité et au niveau de l'extrémité obstruée de la glissière, le bouton-poussoir comprenant alors avantageusement un passage ouvert latéralement que l'ergot est à même d'emprunter pour atteindre la glissière et qui débouche dans la glissière, à distance de la deuxième extrémité de la course maximale. Lorsque tel est le cas, il n'y a aucun risque non plus que le bouton-poussoir commence à se démonter quand il est enfoncé complètement sous l'action d'une pression manuelle et il en résulte une sécurité accrue rendant plus fiable l'utilisation du bouton-poussoir.

Avantageusement, le passage débouchant dans la glissière est formé d'une première partie d'introduction selon la direction de coulissement, et d'une deuxième partie consistant en une rainure latérale qui débouche sur la glissière. De cette façon, l'opération de montage est rendue plus intuitive et plus fiable en dissociant l'opération de couplage mutuel de celle de verrouillage en position de travail par des manipulations distinctes, tout d'abord en translation puis en rotation selon une direction orthogonale. La rainure latérale constitue par ailleurs une sécurité additionnelle en procurant une position intermédiaire entre une position de travail où le coulissement du bouton poussoir est possible et une position de démontage de ce dernier.

Avantageusement, au moins un des passages fait partie de la pièce de retenue et comporte une portion d'entrée qui forme une encoche d'accouplement d'un outil de mise en place de la pièce de retenue dans un logement. De cette manière, cette portion remplit donc une double fonction, à la fois pour le dispositif à baïonnette intrinsèque, et pour le montage de ce dispositif dans la pièce à main.

Avantageusement, la pièce de retenue est annulaire et délimite une ouverture traversante axialement.

Avantageusement, le sous-ensemble comporte un organe élastique de rappel du bouton-poussoir vers l'extérieur.

Avantageusement, la pièce de retenue comporte une portion filetée sur une partie de sa dimension selon la direction de coulissement.

L'invention a également pour objet une pièce à main à usage dentaire ou chirurgical, comportant un trou de réception d'une queue d'outil amovible, ainsi qu'un dispositif de blocage de cette queue d'outil amovible dans le trou. Cette pièce à main comporte un sous-ensemble tel que défini précédemment, le bouton-poussoir étant un bouton d'actionnement du dispositif de blocage.

La pièce à main à usage dentaire ou chirurgical définie ci-dessus peut incorporer une ou plusieurs autres caractéristiques avantageuses, isolément ou en combinaison, en particulier parmi celles précisées ci-après.

Avantageusement, la pièce à main comporte un arbre rotatif associé à des moyens d'entraînement en rotation, cet arbre étant creux pour recevoir la queue d'outil amovible et étant pourvu du dispositif de blocage qui est adapté pour immobiliser la queue d'outil dans et par rapport à l'arbre.

Avantageusement, le sous-ensemble est dans le prolongement de l'arbre rotatif, les moyens d'entraînement en rotation étant configurés pour entraîner l'arbre rotatif dans un premier sens de rotation, la deuxième pièce parmi le bouton-poussoir et la pièce de retenue comportant une entrée/sortie qui donne accès à la glissière, qui peut être empruntée par l'ergot et qui est disposée de manière que l'ergot ne puisse sortir de la glissière que moyennant une rotation du bouton-poussoir dans un deuxième sens de rotation contraire au premier sens de rotation, autour de l'axe, par rapport à la pièce de retenue. Lorsque tel est le cas, l'arbre rotatif entraîné en rotation dans le premier sens de rotation ne peut lui-même entraîner malencontreusement le bouton-poussoir dans le sens d'un démontage.

Avantageusement, la pièce à main comprend un corps d'assemblage dans lequel est fixée la pièce de retenue.

Avantageusement, la pièce à main comprend un corps d'assemblage définissant une tête allongée qui renferme l'arbre et à une extrémité duquel le bouton-poussoir se trouve dans le prolongement de cet arbre.

L'invention a en outre pour objet un procédé de montage d'un sous-ensemble tel que défini précédemment. Ce procédé de montage comprend des étapes dans lesquelles :
b) on positionne le bouton-poussoir par rapport à la pièce de retenue de manière que chaque portion d'accrochage se trouve à une entrée d'un des passages, puis
c) en exécutant une manoeuvre du bouton-poussoir par rapport à la pièce de retenue de manière que cette manœuvre comprenne au moins une composante selon la direction de coulissement et une composante de rotation autour dudit axe, on fait progresser les portions d'accrochage dans les passages jusqu'à amener chaque portion d'accrochage derrière une des butées de fin de course.

Le procédé de montage défini ci-dessus peut incorporer une ou plusieurs autres caractéristiques avantageuses, isolément ou en combinaison, en particulier parmi celles précisées ci-après.

Avantageusement, le procédé de montage comprend une étape qui précède l'étape b) et dans laquelle :
a) on visse la pièce de retenue dans un logement à l'aide d'un outil qui est accouplé à la pièce de retenue par engagement au moins dans une portion d'entrée d'un des passages.

### Brève description des dessins

D'autres avantages et caractéristiques ressortiront plus clairement de la description qui va suivre d'un mode particulier de réalisation de l'invention donné à titre d'exemple non limitatif et représenté aux dessins annexés, parmi lesquels :
- la figure 1 est une vue en coupe axiale d'une tête que comporte une pièce à main de soin dentaire ou de chirurgie selon un mode de réalisation de l'invention ;
- la figure 2 est une vue éclatée, en perspective, d'un sous-ensemble qui est conforme à l'invention et que comporte la pièce à main de soin dentaire ou de chirurgie représentée à la figure 1 ; et
- les figures 3 à 5 sont trois vues analogues en perspective et illustrent chacune l'une de trois étapes successives que comporte l'assemblage du sous-ensemble représenté à la figure 2.

### Description d'un mode de réalisation de l'invention

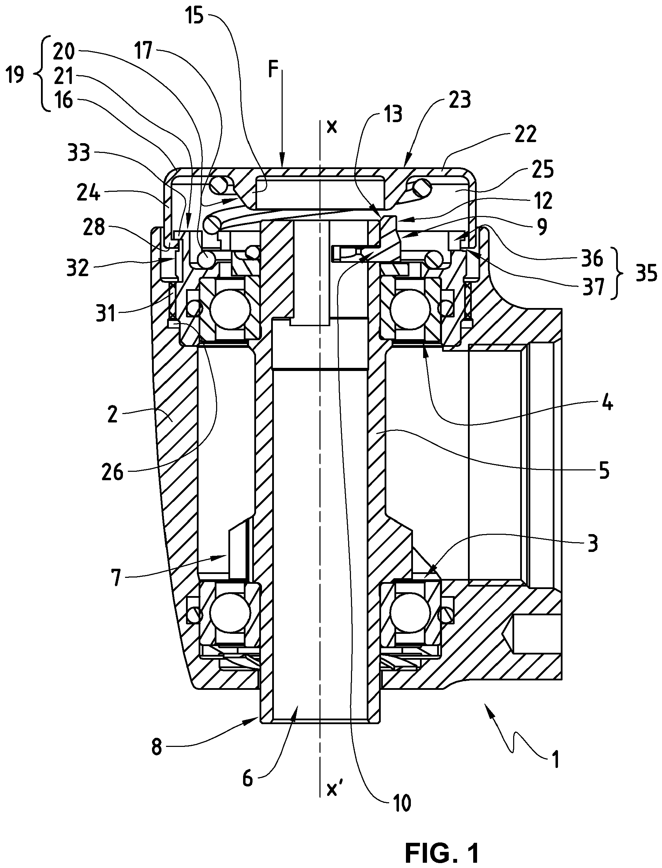

La figure 1 représente partiellement une pièce à main de soin dentaire ou de chirurgie selon un mode de réalisation préférentiel de l'invention. Plus précisément, elle représente une tête 1 que comporte cette pièce à main et qui est destinée à être fixée à une extrémité d'un manche non représenté.

Un corps d'assemblage 2 forme une partie de l'enveloppe extérieure de la tête 1 et renferme deux paliers, à savoir un premier palier 3 et un deuxième palier 4, lesquels supportent ensemble un arbre 5 creux, rotatif sur un axe X-X' et délimitant un trou 6 de réception d'une queue d'outil amovible non représentée. L'arbre 5 comporte une denture annulaire 7 destinée à engrener avec la denture d'un organe non représenté de transmission d'un entraînement.

Parmi les deux extrémités opposées de l'arbre 5, celle référencée 8 est l'extrémité ouverte par laquelle une queue d'outil amovible peut être introduite dans le trou 6. A l'opposé de cette extrémité ouverte 8, l'autre extrémité de l'arbre 5 est pourvue d'un dispositif 9 de blocage de la queue d'outil amovible par rapport à cet arbre 5. Dans l'exemple représenté, le dispositif de blocage 9 comporte un mors de serrage 10 coulissant transversalement par rapport à l'arbre 5 et rappelé élastiquement vers l'axe X-X'.

Le mors de serrage 10 comporte un rebord 12, qui possède un premier chanfrein 13 et qui, grâce à ce dernier, peut être actionné radialement dans la direction opposée à l'axe X-X', par une saillie intérieure 15 d'un bouton-poussoir 16.

La saillie intérieure 15 a la forme d'une nervure annulaire en saillie vers les paliers 3 et 4. Centrée sur l'axe X-X', cette nervure annulaire comporte un deuxième chanfrein 17, qui est prévu pour coopérer avec le premier chanfrein 13 lorsque le bouton-poussoir 16 est actionné manuellement dans le sens de la flèche F, parallèlement à l'axe X-X'.

Le bouton-poussoir 16 fait partie d'un sous-ensemble 19 d'axe X-X', qui comprend également un ressort hélicoïdal 20 et une pièce 21 de retenue du bouton-poussoir 16 vers l'extérieur. Comprimé entre la pièce de retenue 21 et le bouton-poussoir 16, le ressort hélicoïdal 20 forme un organe élastique de rappel de ce bouton-poussoir 16 vers l'extérieur, dans le sens contraire à celui illustré par la flèche F.

Une première portion 22 du bouton-poussoir 16 présente la forme d'un disque dont la face interne porte la saillie intérieure 15 et dont la face externe forme une surface d'appui 23 adaptée pour recevoir une pression manuelle dans le sens de la flèche F. Une deuxième portion du bouton-poussoir 16 est une jupe 24 tubulaire et fermée d'un côté par la première portion 22. Le bouton-poussoir 16 délimite un creux 25 dans lequel pénètre partiellement la pièce de retenue 21.

La pièce de retenue 21 est vissée dans un logement 26 du corps d'assemblage 2. Cette pièce de retenue 21 remplit plusieurs fonctions dans la mesure où elle retient le bouton-poussoir 16 vers l'extérieur et où, en outre, elle bloque le palier 4 latéralement et vers l'extérieur.

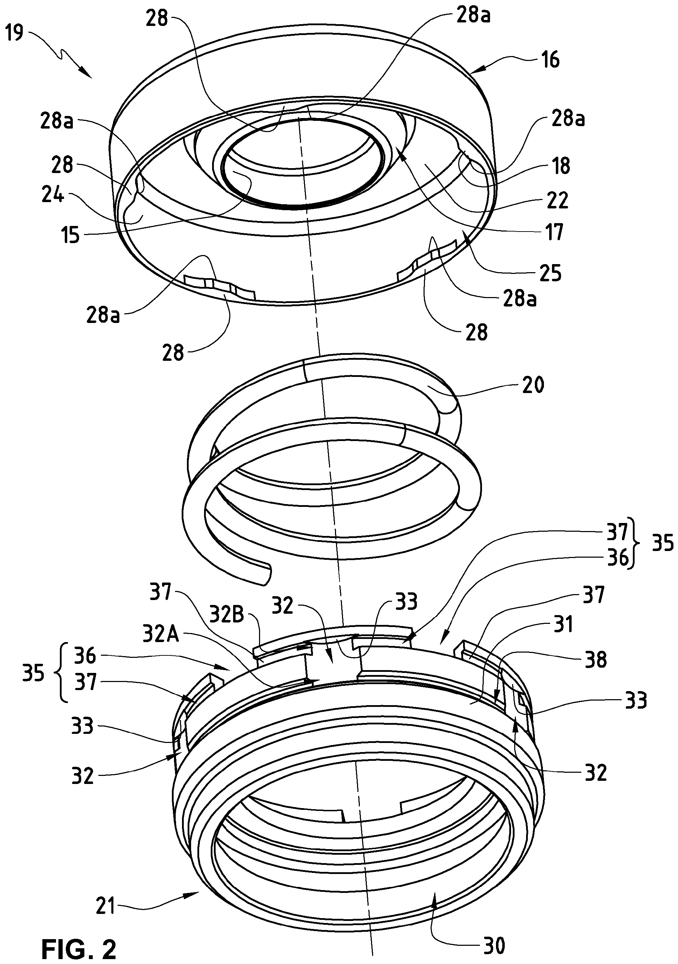

Le sous-ensemble 19 est représenté seul à la figure 2. Ainsi qu'on peut le voir sur cette figure 2, la jupe 24 porte plusieurs ergots intérieurs 28, qui sont en saillie radialement vers l'intérieur et qui sont décalés angulairement entre eux autour de l'axe X-X'. Dans l'exemple représenté, les ergots 28 présentent chacun la forme d'une portion de rebord intérieur.

La pièce de retenue 21 est annulaire et délimite une ouverture traversante axialement 30, dans laquelle est engagée une extrémité de l'arbre 5. La pièce de retenue 21 comporte un bourrelet fileté 31, grâce auquel elle peut être montée par vissage dans le corps d'assemblage 2.

La pièce de retenue 21 comporte également plusieurs gorges axiales, qui s'étendent parallèlement à l'axe X-X' et qui forment des glissières 32 de guidage des ergots 28. Ces glissières 32 sont décalées angulairement entre elles autour de l'axe X-X'. Lorsque chaque ergot 28 est dans une des glissières 32, le bouton-poussoir 16 est guidé par la pièce de retenue 21 de manière à pouvoir coulisser selon une direction de coulissement parallèle à l'axe X-X'. En d'autres termes, la pièce 21 de retenue du bouton-poussoir 16 constitue également une pièce de guidage pour ce bouton-poussoir 16. De cette manière, la fonction de guidage du bouton-poussoir 16 est réalisée de manière autonome par le sous-ensemble lui-même, se dispensant ainsi avantageusement de tout guidage effectué par l'extérieur, via par exemple la correspondance de forme et l'ajustement des dimensions entre la paroi interne du logement 26 et la jupe tubulaire 24. Le sous-ensemble ainsi formé est par conséquent compatible avec différentes têtes 1 dont la taille et les formes du logement prévu pour le bouton-poussoir peuvent légèrement varier.

L'extrémité supérieure de chaque glissière 32 est obstruée selon la direction de coulissement dans cette glissière 32, par une butée de fin de course 33 constituée par une portion de rebord extérieur de la pièce de retenue 21. L'autre extrémité de n'importe quelle glissière 32 se trouve en arrière de l'extrémité obstruée par une des butées de fin de course 33. Elle est elle-même obstruée selon la direction de coulissement, par le bourrelet fileté 31. De la sorte, une fois dans les glissières 32, les ergots 28 ont chacun une course maximale limitée par le bourrelet fileté 31, constituant une première extrémité 32A pour la glissière 32, et l'une des butées de fin de course 33, constituant la deuxième extrémité 32B pour la glissière 32.

Des passages 35 ouverts latéralement mènent aux glissières 32, depuis l'avant de la pièce de retenue 21. Chaque passage 35 comporte une encoche 36 d'entrée et une rainure latérale. Selon le mode de réalisation préférentiel illustré sur les figures 2 à 5, la rainure latérale est une première rainure latérale 37 qui débouche dans une glissière 32, à distance de son extrémité obstruée par le bourrelet fileté 31. Chaque rainure latérale 37 forme une entrée/sortie qui donne accès à une glissière. Chaque ergot 28 peut emprunter un des passages 35 pour venir derrière une des butées de fin de course 33. On peut toutefois noter qu'une deuxième rainure latérale 38 relie également les extrémités inférieures de chacune des glissières 32, c'est-à-dire les premières extrémités 32A de ces dernières.

La pièce de retenue 21 et le bouton-poussoir 16 sont configurés d'une manière complémentaire telle que lorsque les ergots 28 sont dans les passages 35, une manœuvre du bouton-poussoir par rapport à la pièce de retenue 21 peut être effectuée et comprend au moins une composante selon la direction de coulissement parallèle à l'axe X-X' et une composante de rotation autour de cet axe X-X'. Les ergots 28 et les butées de fin de course 33 font partie d'un dispositif à baïonnette à même de maintenir assemblés la pièce de retenue 21 et le bouton-poussoir 16. En ce sens, chaque ergot 28 forme une portion d'accrochage 28a qu'une butée de fin de course 33 est à même de retenir vers l'extérieur, à l'encontre de la poussée exercée par le ressort hélicoïdal 20 dans le sens contraire de la flèche F.

Pour monter le sous-ensemble 19 dans le corps d'assemblage 2, on commence par visser la pièce de retenue 21 seule, c'est-à-dire dépourvue du bouton-poussoir 16, dans ce corps d'assemblage 2. Pour ce faire, on peut utiliser un outil non représenté en prise avec les bords des encoches 36, qui forment des encoches d'accouplement d'un tel outil en plus d'être des portions d'entrée des passages ouverts latéralement 35.

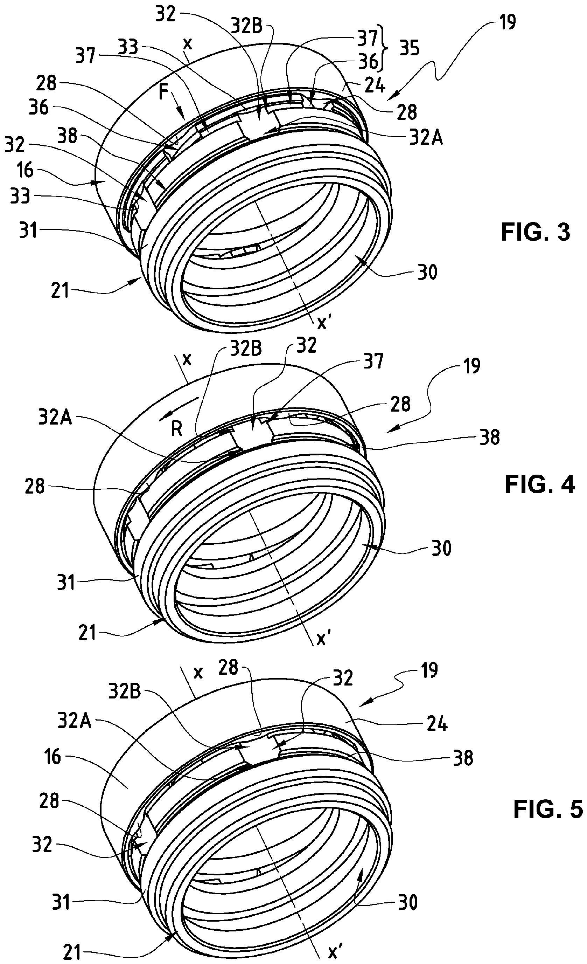

Ensuite, on monte le bouton-poussoir 16 sur la pièce de retenue 21. Pour ce faire, on commence par placer le bouton-poussoir 16 devant la pièce de retenue 21, comme il l'est à la figure 3, c'est-à-dire de manière que chaque ergot 28 se trouve au niveau d'une des encoches 36. Ensuite, on pousse le bouton-poussoir 16 dans la direction de la flèche F, jusqu'à ce que chaque ergot ait atteint le fond d'une des encoches 36 et se trouve à la même profondeur que les premières rainures latérales 37.

La manœuvre suivante consiste à faire pivoter le bouton-poussoir 16 par rapport à la pièce de retenue 21, autour de l'axe X-X'. Elle est symbolisée par la flèche R à la figure 4, matérialisant une rotation dans le sens anti-horaire. Lorsque chaque ergot 28 a atteint une des glissières 32 et se trouve derrière une des butées de fin de course 33, le montage du sous-ensemble 19 est terminé.

Une fois en place sur la pièce de retenue 21, le bouton-poussoir 16 est démontable. Pour ôter le bouton-poussoir 16 de la pièce de retenue 21, on applique la procédure inverse de celle décrite ci-dessus, qui est à employer pour monter le bouton-poussoir 16 sur la pièce de retenue 21.

On comprendra, au vu de ce qui précède, que l'invention ne se limite pas au mode de réalisation préférentiel décrit précédemment, qui n'a été donné qu'à titre d'exemple, sans avoir pour vocation d'être exhaustif pour en définir le champ de de protection. En particulier, les ergots 28 pourraient également être portés par la pièce de retenue 21 et former les butées de fin de course, tandis que les glissières 32 pourraient être portées par la jupe 24 du bouton-poussoir 16.

De plus, la pièce de retenue 21 et le corps d'assemblage 2 peuvent également ne pas former deux pièces distinctes, mais être combinés sous la forme d'une seule pièce monobloc, c'est-à-dire d'un seul tenant.

Par ailleurs, la jupe 24 pourrait également être moins large que l'ouverture 30, auquel cas les ergots 28, les glissières 32 et les butées de fin de course 33 peuvent se trouver dans cette ouverture 30.

En outre, selon une variante, il est possible d'envisager soit de supprimer les deuxièmes rainures latérales 38, soit de n'utiliser que celles-ci en lieu et places des premières rainures latérales 37 pour communiquer directement avec les encoches 36 et former les passages 35, débouchant ainsi non plus sur la glissière 32 au niveau de sa deuxième extrémité 32B, mais au niveau de sa première extrémité 32A. Toutefois, si l'opération de montage du bouton-poussoir 16 n'en est pas affectée, un démontage intempestif de ce dernier ne serait alors pas exclu lorsqu'il est actionné.

Selon une autre variante, chaque passage 35 débouche dans une glissière 32 à distance des première et deuxième extrémités 32A et 32B de celle-ci, entre ces première et deuxième extrémités 32A et 32B, c'est-à-dire ni au niveau de l'une et ni au niveau de l'autre. Cela réduit encore plus le risque d'un démontage malencontreux du bouton-poussoir 16.

Selon encore une autre variante, une seule rainure latérale 37 débouche dans une glissière 32. Elle forme alors l'unique entrée/sortie qu'un ergot 28 peut emprunter pour entrer dans cette glissière 32 ou en sortir. Lorsque tel est le cas, un ergot 28 ne peut sortir de la glissière 32 à une seule entrée/sortie que moyennant une rotation du bouton-poussoir 16 dans un sens de rotation prédéfini autour de l'axe X-X', par rapport à la pièce de retenue 21. Ce sens de rotation prédéfini est avantageusement choisi de manière à être contraire au sens de rotation dans lequel l'arbre rotatif 5 est entrainé par les moyens d'entraînement en rotation. De la sorte, l'arbre rotatif 5 entraîné en rotation ne peut pas provoquer un démontage du bouton-poussoir 16.

Enfin, le dispositif de blocage peut posséder n'importe quelle constitution appropriée, laquelle peut être différente de celle décrite précédemment. Par exemple, ce dispositif de blocage peut être l'un de ceux des pièces à main décrites dans les demandes de brevet EP 1 378 207, DE 432 44 93 et EP 1 733 696 mentionnées dans l'introduction.

## Revendications

1. Sous-ensemble pour pièce à main de soin dentaire ou de chirurgie, comportant:
- un bouton-poussoir (16) comprenant une surface d'appui (23) adaptée pour recevoir une pression manuelle à même d'actionner le bouton-poussoir (16) en coulissement selon une direction de coulissement, et
- une pièce (21) de retenue du bouton-poussoir (16),
**caractérisé en ce qu'**il comprend un dispositif à baïonnette (28a, 33, 35) qui est à même de maintenir assemblés le bouton-poussoir (16) et la pièce de retenue (21) et dans lequel :
- le bouton-poussoir (16) comporte plusieurs portions d'accrochage (28a) décalées angulairement entre elles autour d'un axe (X-X') parallèle à la direction de coulissement, et
- la pièce de retenue (21) comporte plusieurs butées de fin de course (33) à même de stopper les portions d'accrochage (28a) vers l'extérieur, ainsi que plusieurs passages (35) ouverts latéralement qui passent entre les butées de fin de course (33), qui mènent derrière ces butées de fin de course (33) et que les portions d'accrochage (28a) sont à même d'emprunter pour venir derrière les butées de fin de course (33).

2. Sous-ensemble selon la revendication 1, **caractérisé en ce que** le bouton-poussoir (16) comporte une première portion (22) où se trouve la surface d'appui (23), le bouton-poussoir (16) comportant une deuxième portion qui est une jupe (24) fermée d'un côté par la première portion (22), la première portion (22) et la jupe (24) délimitant un creux (25) dans lequel pénètre au moins une partie de la pièce de retenue (21) et dans lequel se trouvent les butées de fin de course (33) et les portions d'accrochage (28a).

3. Sous-ensemble selon l'une quelconque des revendications précédentes, **caractérisé en ce que** la pièce de retenue (21) est également une pièce de guidage du bouton-poussoir (16) selon la direction de coulissement.

4. Sous-ensemble selon l'une quelconque des revendications précédentes, **caractérisé en ce qu'**il comporte au moins un ergot (28) que porte une première pièce parmi le bouton-poussoir (16) et la pièce de retenue (21), la deuxième pièce parmi le bouton-poussoir (16) et la pièce de retenue (21) comportant au moins une glissière (32) de guidage de l'ergot (28) selon la direction de coulissement.

5. Sous-ensemble selon la revendication 4, **caractérisé en ce que** la pièce de retenue (21) comporte la glissière (32) qui possède une extrémité obstruée par une des butées de fin de course (33), **en ce qu'**un des passages (35) faisant partie de la pièce de retenue (21) débouche dans la glissière (32), et **en ce que** l'ergot (28) fait partie du bouton-poussoir (16), forme une des portions d'accrochage (28a) et est à même d'emprunter le passage (35) débouchant dans la glissière (32).

6. Sous-ensemble selon la revendication 4, **caractérisé en ce que** la pièce de retenue (21) comporte la glissière (32) qui possède une extrémité obstruée par une des butées de fin de course (33), l'ergot (28) étant mobile en translation dans la glissière (32) entre une première extrémité (32A) et une deuxième extrémité (32B) opposée se trouvant au-dessus de la première extrémité (32A) et située au niveau de l'extrémité obstruée de la glissière (32), un des passages (35) faisant partie de la pièce de retenue (21) et débouchant dans la glissière (32), à distance de la première extrémité (32A) de la glissière (32), tandis que l'ergot (28) fait partie du bouton-poussoir (16), forme une des portions d'accrochage (28a) et est à même d'emprunter le passage (35) débouchant dans la glissière (32).

7. Sous-ensemble selon la revendication 4, **caractérisé en ce que** la pièce de retenue (21) comporte la glissière (32) qui possède une extrémité obstruée par une des butées de fin de course (33), l'ergot (28) étant mobile en translation dans la glissière (32) entre une première extrémité (32A) et une deuxième extrémité (32B) opposée se trouvant au-dessus de la première extrémité (32A) et située au niveau de l'extrémité obstruée de la glissière (32), un des passages (35) faisant partie de la pièce de retenue (21) et débouchant dans la glissière (32), à distance de la première extrémité (32A) et de la deuxième extrémité (32B) de la glissière (32), tandis que l'ergot (28) fait partie du bouton-poussoir (16), forme une des portions d'accrochage (28a) et est à même d'emprunter le passage (35) débouchant dans la glissière (32).

8. Sous-ensemble selon l'une des revendications 5 à 7, **caractérisé en ce que** le passage (35) débouchant dans la glissière (32) est formé d'une première partie d'introduction selon la direction de coulissement, ainsi que d'une deuxième partie consistant en une rainure latérale (37,38) débouchant dans la glissière (32).

9. Sous-ensemble selon la revendication 4, **caractérisé en ce que** l'ergot fait partie de la pièce de retenue et forme une des butées de fin de course, le bouton-poussoir comprenant la glissière.

10. Sous-ensemble selon l'une quelconque des revendications précédentes, **caractérisé en ce qu'**au moins un des passages (35) fait partie de la pièce de retenue (21) et comporte une portion d'entrée qui forme une encoche (36) d'accouplement d'un outil de mise en place de la pièce de retenue (21) dans un logement (26).

11. Sous-ensemble selon l'une quelconque des revendications précédentes, **caractérisé en ce qu'**il comporte un organe élastique (20) de rappel du bouton-poussoir (16) vers l'extérieur.

12. Pièce à main à usage dentaire ou chirurgical, comportant un trou (6) de réception d'une queue d'outil amovible, ainsi qu'un dispositif (9) de blocage de cette queue d'outil amovible dans le trou (6),
**caractérisée en ce qu'**elle comporte un sous-ensemble (19) selon l'une quelconque des revendications précédentes, le bouton-poussoir (16) étant un bouton d'actionnement du dispositif de blocage (9).

13. Pièce à main selon la revendication 12, **caractérisée en ce qu'**elle comporte un arbre rotatif (5) associé à des moyens d'entraînement en rotation, cet arbre (5) étant creux pour recevoir la queue d'outil amovible et étant pourvu du dispositif de blocage (9) qui est adapté pour immobiliser la queue d'outil dans et par rapport à l'arbre (5).

14. Pièce à main selon la revendication 13, **caractérisée en ce que** le sous-ensemble (19) est selon l'une des revendications 4 à 9, ce sous-ensemble (19) étant dans le prolongement de l'arbre rotatif (5), les moyens d'entraînement en rotation étant configurés pour entraîner l'arbre rotatif (5) dans un premier sens de rotation, la deuxième pièce parmi le bouton-poussoir (16) et la pièce de retenue (21) comportant une entrée/sortie (37) qui donne accès à la glissière (32), qui peut être empruntée par l'ergot (28) et qui est disposée de manière que l'ergot (28) ne puisse sortir de la glissière (32) que moyennant une rotation du bouton-poussoir (16) dans un deuxième sens de rotation contraire au premier sens de rotation, autour de l'axe (X-X'), par rapport à la pièce de retenue (21).

15. Procédé de montage d'un sous-ensemble (19) selon l'une quelconque des revendications 1 à 11, comprenant des étapes dans lesquelles :
b) on positionne le bouton-poussoir (16) par rapport à la pièce de retenue (21) de manière que chaque portion d'accrochage (28a) se trouve à une entrée d'un des passages (35), puis
c) en exécutant une manœuvre du bouton-poussoir (16) par rapport à la pièce de retenue (21) de manière que cette manœuvre comprenne au moins une composante selon la direction de coulissement et une composante de rotation autour dudit axe (X-X'), on fait progresser les portions d'accrochage (28a) dans les passages (35) jusqu'à amener chaque portion d'accrochage (28a) derrière une des butées de fin de course (33).

## Patentansprüche

1. Untergruppe für eine zahnärztliches oder chirurgisches Handstück, umfassend:
- eine Drucktaste (16), umfassend eine Kontaktfläche (23), welche eingerichtet ist, um einen manuellen Druck aufzunehmen, um die Drucktaste (16) schiebend in einer Schieberichtung zu aktivieren,
- ein Element (21), um die Drucktaste (16) zu halten,
**dadurch gekennzeichnet, dass** sie eine Bajonett-Vorrichtung (28a, 33, 35) umfasst, welche die Drucktaste (16) und das Halteelement (21) in zusammengesetzter Anordnung halten kann und wobei:
- die Drucktaste (16) eine Vielzahl von Eingriffsabschnitten (28a) aufweist, welche um eine Achse (X-X') parallel zu der Schieberichtung winkelig zueinander versetzt sind, und
- das Halteelement (21) eine Vielzahl von Anschlägen (33) umfasst, welche die Eingriffsabschnitte (28a) bezogen auf die Aussenseite halten können, sowie eine Vielzahl von lateralen offenen Durchgängen (35), welche zwischen den Anschlägen (33) verlaufen, und welche über die Anschläge (33) hinaus gehen und die Eingriffsabschnitte (28a) nachfolgen können, so dass diese hinter die Anschläge (33) gelangen.

2. Untergruppe nach Anspruch 1, **dadurch gekennzeichnet, dass** die Drucktaste (16) einen ersten Abschnitt (22) umfasst, in dem die Kontaktfläche (23) lokalisiert ist, wobei die Drucktaste (16) einen zweiten Abschnitt umfasst, welcher eine Seitenwand (24) ist, die an einer Seite durch den ersten Abschnitt (22) geschlossen ist, der erste Abschnitt (22) und die Seitenwand (24) einen Hohlraum (25) begrenzen, in welchen mindestens Teile des Halteelements (21) eindringen und in welchen sich die Anschläge (33) und die Eingriffsabschnitte (28a) befinden.

3. Untergruppe nach einem der vorhergehenden Ansprüche, **dadurch gekennzeichnet, dass** das Halteelement (21) auch ein Element ist, um die Drucktaste (16) in der Schieberichtung zu führen.

4. Untergruppe nach einem der vorhergehenden Ansprüche, **dadurch gekennzeichnet, dass** sie mindestens eine Nase (28) umfasst, welche ein erstes Teil zwischen der Drucktaste (16) und dem Halteelement (21) trägt, wobei der zweite Teil zwischen der Drucktaste (16) und dem Halteelement (21) mindestens eine Führungsschiene (32) umfasst, um die Nase (28) in der Schieberichtung zu führen.

5. Untergruppe nach Anspruch 4, **dadurch gekennzeichnet, dass** das Halteelement (21) die Führungsschiene (32) umfasst, die ein Ende aufweist, das von einem der Anschläge (33) blockiert ist, dass eines der Durchgänge (35), welche einen Teil des Halteelements (21) bilden, in der Führungsschiene (32) mündet und dadurch dass die Nase (28) einen Teil der Drucktaste (16) bildet, eine der Eingriffsabschnitte (28a) bildet und dem Durchgang (35) nachfolgen kann, welcher in der Führungsschiene (32) mündet.

6. Untergruppe nach Anspruch 4, **dadurch gekennzeichnet, dass** das Halteelement (21) die Führungsschiene (32) umfasst, die ein Ende aufweist, das von einem der Anschläge (33) blockiert ist, die Nase (28) in der Führungsschiene zwischen einem ersten Ende (32A) und einem gegenüberliegenden zweiten Ende (32B) verschieblich bewegbar ist, welches oberhalb des ersten Endes (32A) angeordnet ist und an dem blockierten Ende der Führungsschiene (32) liegt, einer der Durchgänge (35) einen Teil des Halteelements (21) bildet und in der Führungsschiene (32) in einem Abstand zu dem ersten Ende (32A) der Führungsschiene (32) mündet, während die Nase (28) einen Teil der Drucktaste (16) bildet, einen der Eingriffsabschnitte (28a) bildet und dem Durchgang (35) nachfolgen kann, welcher in der Führungsschiene (32) mündet

7. Untergruppe nach Anspruch 4, **dadurch gekennzeichnet, dass** das Halteelement (21) die Führungsschiene (32) umfasst, die ein Ende aufweist, welches von einem der Anschläge (33) blockiert ist, die Nase (28) in der Führungsschiene (32) zwischen einem ersten Ende (32A) und einem gegenüberliegenden zweiten Ende (32B) verschieblich bewegbar ist, welches oberhalb des ersten Endes (32A) angeordnet ist und an dem blockierten Ende der Führungsschiene (32) liegt, einer der Durchgänge (35) einen Teil des Halteelements (21) bildet und in der Führungsschiene (32) in einem Abstand zu dem ersten Ende (32A) und zu dem zweiten Ende (32B) der Führungsschiene (32) mündet, während die Nase (28) einen Teil der Drucktaste (16) bildet, einen der Eingriffsabschnitte (28a) bildet und dem Durchgang (35) nachfolgen kann, welcher in der Führungsschiene (32) mündet.

8. Untergruppe nach einem der Ansprüche 5 bis 7, **dadurch gekennzeichnet, dass** der Durchgang (35), der in der Führungsschiene (32) mündet, von einem ersten Einführungsteil in der Schieberichtung gebildet ist, sowie einem zweiten Teil, welcher von einer seitlichen Nut (37, 38) gebildet ist, welche in der Führungsschiene (32) mündet.

9. Untergruppe nach Anspruch 4, **dadurch gekennzeichnet, dass** die Nase einen Teil des Halteelements bildet und einen der Anschläge bildet, wobei die Drucktaste die Führungsschiene umfasst.

10. Untergruppe nach einem der vorhergehenden Ansprüche, **dadurch gekennzeichnet, dass** einer der Durchgänge (35) einen Teil des Halteelements (21) bildet und einen Eingangsabschnitt umfasst, welcher einen Kupplungsschlitz (36) für ein Werkzeug bildet, um das Halteelement (21) in einer Aufnahme (26) zu platzieren.

11. Untergruppe nach einem der vorhergehenden Ansprüche, **dadurch gekennzeichnet, dass** sie ein elastisches Element (20) umfasst, um die Drucktaste (16) nach aussen zurückzusetzen.

12. Handstück für zahnärztlichen oder chirurgischen Gebrauch, umfassend eine Öffnung (6), um das Endstück eines entfernbaren Werkzeugs aufzunehmen, sowie eine Vorrichtung (9), um das Endstück des entfernbaren Werkzeugs in der Öffnung (6) zu arretieren,
**dadurch gekennzeichnet, dass** es eine Untergruppe (19) nach einem der vorhergehenden Ansprüche umfasst, wobei die Drucktaste (16) eine Taste zum Aktivieren der Verriegelungsvorrichtung (9) ist.

13. Handstück nach Anspruch 12, **dadurch gekennzeichnet, dass** es eine Drehwelle (5) umfasst, welche mit Drehantriebsmitteln verbunden ist, wobei die Welle (5) hohl ist, um das Endstück eines entfernbaren Werkzeugs aufzunehmen und mit einer Verriegelungsvorrichtung (9) versehen ist, welche eingerichtet ist, um das Endstück des Werkzeugs in der, und bezogen auf, die Welle (5) festzulegen.

14. Handstück nach Anspruch 13, **dadurch gekennzeichnet, dass** die Untergruppe (19) eine Untergruppe nach einem der Ansprüche 4 bis 9 ist, wobei die Untergruppe (19) in der Verlängerung der Drehwelle (5) ist, wobei die Mittel zum Drehantrieb eingerichtet sind, um die Drehwelle (5) in einer ersten Drehrichtung anzutreiben, das zweite Element zwischen der Drucktaste (16) und dem Halteelement (21) einen Eingang/Ausgang (37) umfasst, welcher Zugang zu der Führungsschiene (32) bereitstellt, welchem die Nase (28) nachfolgen kann und welcher so angeordnet ist, dass die Nase (28) nur die Führungsschiene (32) verlassen kann mittels einer Drehung der Drucktaste (16) in einer entgegengesetzt zu der ersten Drehrichtung zweiten Drehrichtung um die Achse (X-X'), bezogen auf das Halteelement (21).

15. Verfahren zum Zusammenbau einer Untergruppe (19) gemäss einem der Ansprüche 1 bis 11, umfassend die Schritte, welche sind:
b) Positionieren der Drucktaste (16) bezogen auf das Halteelement (21), so dass jeder Eingriffsabschnitt (28a) an einem Eingang einer der Durchgänge (35) angeordnet ist, dann
c) Beim Durchführen einer Handhabung der Drucktaste (16) bezogen auf das Halteelement (21) derart, dass die Handhabung mindestens eine Komponente in der Schieberichtung und eine Drehkomponente um die Achse (X-X') enthält, kommen die Eingriffsabschnitte (28a) in den Durchgängen (35) solange weiter, bis jeder Eingriffsabschnitt (28a) hinter einen der Anschläge (33) gebracht ist.

## Claims

1. Subassembly for a dental care or surgical handpiece, comprising:
- a push button (16) comprising a contact surface (23) adapted to receive a manual pressure able to actuate the push button (16) slidably in a sliding direction, and
- a piece (21) for retaining the push button (16),
**characterized in that** it comprises a bayonet device (28a, 33, 35) which is able to keep assembled the push button (16) and the retaining piece (21) and wherein:
- the push button (16) comprises a plurality of engagement portions (28a) angularly offset with respect to one another about an axis (X-X') parallel to the direction of sliding, and
- the retaining piece (21) comprises a plurality of limit stops (33) able to retain the engagement portions (28a) with respect to the outside, as well as a plurality of laterally open passages (35) which pass between the limit stops (33), which run beyond these limit stops (33), and the engagement portions (28a) are able to follow so as to come behind the limit stops (33).

2. Subassembly according to claim 1, **characterized in that** the push button (16) comprises a first portion (22) where the contact surface (23) is located, the push button (16) comprising a second portion which is a lateral wall (24) closed on one side by the first portion (22), the first portion (22) and the lateral wall (24) delimiting a cavity (25) into which penetrates at least part of the retaining piece (21) and in which are found the limit stops (33) and the engagement portions (28a).

3. Subassembly according to any one of the preceding claims, **characterized in that** the retaining piece (21) is also a piece for guiding the push button (16) in the sliding direction.

4. Subassembly according to any one of the preceding claims, **characterized in that** it comprises at least one lug (28) that a first piece between the push button (16) and the retaining piece (21) carries, the second piece between the push button (16) and the retaining piece (21) comprising at least one slide (32) for guiding the lug (28) in the sliding direction.

5. Subassembly according to claim 4, **characterized in that** the retaining piece (21) comprises the slide (32) which has an end blocked by one of the limit stops (33), **in that** one of the passages (35) forming part of the retaining piece (21) comes out into the slide (32), and **in that** the lug (28) forms part of the push button (16), forms one of the engagement portions (28a) and is able to follow the passage (35) coming out into the slide (32).

6. Subassembly according to claim 4, **characterized in that** the retaining piece (21) comprises the slide (32) which has an end blocked by one of the limit stops (33), the lug (28) being movable in translation in the slide (32) between a first end (32A) and an opposite second end (32B) being located above the first end (32A) and situated at the blocked end of the slide (32), one of the passages (35) forming part of the retaining piece (21) and coming out into the slide (32), at a distance from the first end (32A) of the slide (32), while the lug (28) forms part of the push button (16), forms one of the engagement portions (28a) and is able to follow the passage (35) coming out into the slide (32).

7. Subassembly according to claim 4, **characterized in that** the retaining piece (21) comprises the slide (32) which has an end blocked by one of the limit stops (33), the lug (28) being movable in translation in the slide (32) between a first end (32A) and an opposite second end (32B) being located above the first end (32A) and situated at the blocked end of the slide (32), one of the passages (35) forming part of the retaining piece (21) and coming out into the slide (32), at a distance from the first end (32A) and from the second end (32B) of the slide (32), while the lug (28) forms part of the push button (16), forms one of the engagement portions (28a) and is able to follow the passage (35) coming out into the slide (32).

8. Subassembly according to one of the claims 5 to 7, **characterized in that** the passage (35) coming out into the slide (32) is formed by a first introduction part in the direction of sliding, as well as a second part consisting of a lateral groove (37,38) coming out into the slide (32).

9. Subassembly according to claim 4, **characterized in that** the lug forms part of the retaining piece and forms one of the limit stops, the push button comprising the slide.

10. Subassembly according to any one of the preceding claims, **characterized in that** at least one of the passages (35) forms part of the retaining piece (21) and comprises an entry portion which forms a coupling slot (36) for a tool for putting the retaining piece (21) in place in an accommodation (26).

11. Subassembly according to any one of the preceding claims, **characterized in that** it comprises a resilient member (20) for returning the push button (16) outwardly.

12. Handpiece for dental or surgical use, comprising a hole (6) for receiving the tail end of a removable tool, as well as a device (9) for locking this tail end of the removable tool in the hole (6),
**characterized in that** it comprises a subassembly (19) according to any one of the preceding claims, the push button (16) being a button for actuation of the locking device (9).

13. Handpiece according to claim 12, **characterized in that** it comprises a rotary shaft (5) connected to means of driving in rotation, this shaft (5) being hollow to receive the tail end of a removable tool and being provided with a locking device (9) which is designed to immobilize the tail end of the tool in, and with respect to, the shaft (5).

14. Handpiece according to claim 13, **characterized in that** the subassembly (19) is according to one of the claims 4 to 9, this subassembly (19) being in the prolongation of the rotary shaft (5), the means of driving in rotation being configured to drive the rotary shaft (5) in a first direction of rotation, the second piece between the push button (16) and the retaining piece (21) comprising an entry/exit (37) which gives access to the slide (32), which can be followed by the lug (28) and which is disposed in such a way that the lug (28) is only able to leave the slide (32) by means of a rotation of the push button (16) in a second direction of rotation opposite the first direction of rotation, about the axis (X-X'), with respect to the retaining piece (21).

15. Method of assembly of a subassembly (19) according to any one of the claims 1 to 11, comprising the steps which are:
b) positioning the push button (16) with respect to the retaining piece (21) in such a way that each engagement portion (28a) is located at an entry of one of the passages (35), then
c) by executing a manoeuvre of the push button (16) with respect to the retaining piece (21) in such a way that this manoeuvre includes at least a component in the direction of sliding and a component of rotation about the said axis (X-X'), making the engagement portions (28a) progress in the passages (35) until each engagement portion (28a) is brought beyond one of the limit stops (33).
